Europäisches Patentamt

European Patent Office

Office européen des brevets

(11) Publication number: **0 492 247 A1**

# EUROPEAN PATENT APPLICATION

(21) Application number: **91121064.9**

(22) Date of filing: **09.12.91**

(51) Int. Cl.5: **A61K 31/34**, A61K 9/00

(30) Priority: **21.12.90 US 633230**

(43) Date of publication of application:
**01.07.92 Bulletin 92/27**

(84) Designated Contracting States:
**AT BE CH DE DK ES FR GB GR IT LI LU NL SE**

(71) Applicant: **MILES INC.**
**1127 Myrtle Street**
**Elkhart,IN 46515(US)**

(72) Inventor: **Paulos, Manley A.**
**15626 Cold Springs Court**
**Granger, Indiana 46530(US)**

(74) Representative: **Dänner, Klaus, Dr. et al**
**c/o Bayer AG Konzernverwaltung RP**
**Patentabteilung**
**W-5090 Leverkusen 1 Bayerwerk(DE)**

(54) **Effervescent H2 blocker formulation.**

(57) An aqueous solution of $H_2$ Blocker and an effervescent delivery system. Immediate and continuous relief is provided for from about 1 to about 3 hours, even when the administered composition contains less than about 25 percent of the dosage of $H_2$ Blocker prescribed for active duodenal ulcer.

FIG.1a

FIG.1d

## Background of the Invention

Histamine $H_2$ receptor blocking agents (referred to herein as $H_2$ Blockers) are a class of drugs which act as antagonists of the histamine $H_2$ receptor. $H_2$ Blockers are currently sold as prescription drugs in the United States for the treatment of medical conditions such as active duodenal ulcer, and pathological hypersecretory conditions such as Zollinger-Ellison syndrome and systemic mastocytosis. Some $H_2$ Blockers have also been approved for the prophylactic treatment of reflux esophagitis. $H_2$ Blockers are effective means of inhibiting gastric acid secretion. Such compounds have a delayed onset, generally one to two hours after ingestion, and a long duration of action. However, these medical conditions are considered severe enough to require the marked inhibition of gastric acid secretion over a prolonged period.

Effervescent formulations such as ALKA-SELTZER® effervescent tablets (Miles Inc., Elkhart, IN, USA), are known to function as antacids by neutralizing gastric acid. Such formulations are administered as solutions and have an immediate onset and short duration.

US Patent 4,824,664, assigned to Smith, Kline & French, discloses an effervescent $H_2$ Blocker formulation prepared with a citric acid and at least a stoichiometric amount of alkali metal carbonate or bicarbonate. The specification notes that $H_2$ Blockers in general are useful in the treatment of ulcerations of gastroduodenal mucosa (duodenal ulcer, benign gastric ulcer, recurrent ulcer and post surgical peptic ulcer), peptic-esophagitis, hemorrhages resulting from esophageal, gastric or duodenal ulcerations or erosions and Zollinger-Ellison syndrome. It is also stated that the effervescent compositions containing an alkali metal bicarbonate do not modify the bioavailability of the $H_2$ antagonist but would allegedly provide the acceleration of its absorption. Tablets containing 800 mg of cimetidine (effervescent and classical tablet formulations) were administered to patients and plasma levels monitored. It is stated that the effervescent compositions of the invention are characterized by particularly good stability during storage, complementary antacid potential and could contain significant amounts of antacid, such as aluminum or magnesium hydroxide or an alkali metal or alkali earth metal carbonate or bicarbonate. In the examples, effervescent tablets containing 100 milligrams (mg) cimetidine were used to measure stability.

GB 2,219,940 A, a British patent application assigned to Glaxo, discloses a particular effervescent formulation of ranitidine or a physiologically acceptable salt thereof, based on the use of an alkaline carbonate or bicarbonate and solely a monoalkali metal citrate as the effervescent couple. It is stated that ranitidine is a potent histamine $H_2$ antagonist which is widely used in the treatment of conditions where there is an advantage in lowering gastric acidity. Mentioned are such conditions as duodenal and gastric ulceration, reflux oesophagitis and Zollinger-Ellison syndrome. It is also stated that ranitidine may also be used prophylactically in surgical procedures and in the treatment of allergic and inflammatory conditions where histamine is a known mediator. The '940 application states that "the amount of ranitidine, preferably in the form of a physiologically acceptable salt, employed in the effervescent composition of the invention may be for example 50 - 600 mg, and will preferably be in the range of 50 - 500 mg, more preferably 150-300 mg per dosage unit." The examples disclose the use of 150 and 300 mg ranitidine per tablet.

The Physicians' Desk Reference (PDR), 44th Edition, 1990 (page 1013, Col. 1, Indications and Usage) states that Zantac (ranitidine) may be used for short-term treatment of active duodenal ulcer, maintenance therapy for duodenal ulcer, treatment of pathological hypersecretory conditions, short-term treatment of active, benign gastric ulcer, and treatment of gastroesophageal reflux disease.

There has been no recognition to date that the use of an effervescent delivery system with an $H_2$ Blocker would provide a dosage form suitable for self-medication by consumers for the relief of the symptoms of upset stomach associated with heartburn, sour stomach or acid indigestion.

## Summary of the Invention

The invention provides a method of treating an individual suffering from upset stomach associated with heartburn, sour stomach or acid indigestion, by dissolving in water a composition comprising a water soluble $H_2$ Blocker and an effervescent delivery system, and orally administering the resulting solution to the individual, thereby providing immediate and continuous relief for from about 1 to about 3 hours. A composition for treating such an individual is also provided, composed of a water soluble $H_2$ Blocker and an effervescent delivery system, the amount of $H_2$ Blocker being less than about 25 percent of the dosage prescribed for twice daily treatment of duodenal ulcer, the composition providing immediate and continuous relief for from about 1 to about 3 hours when dissolved in water and administered orally.

## Brief Description of the Drawings

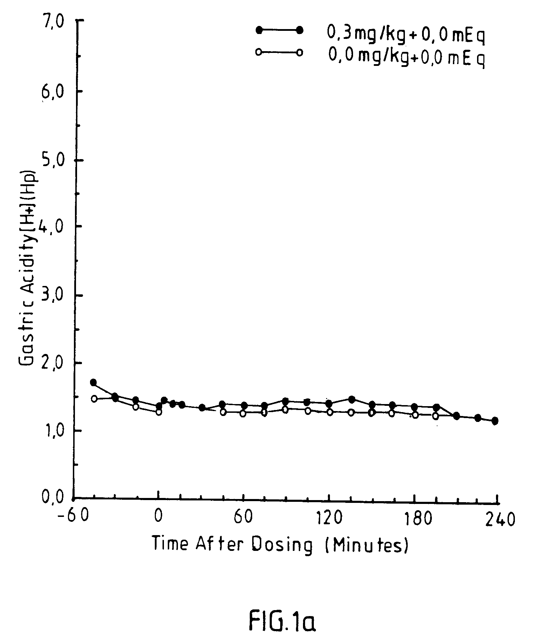

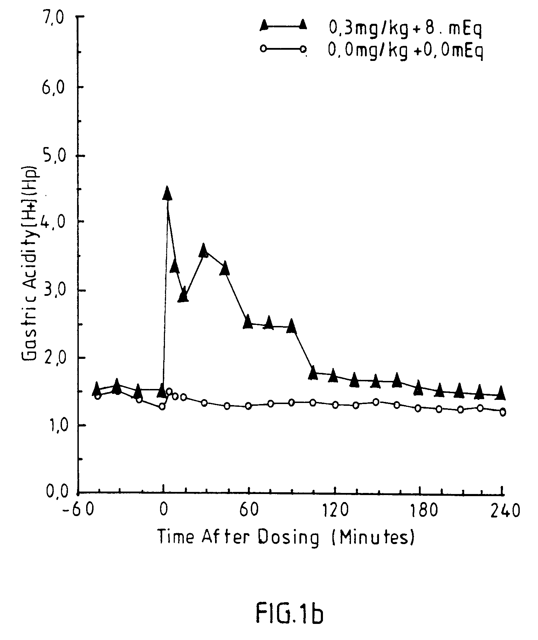

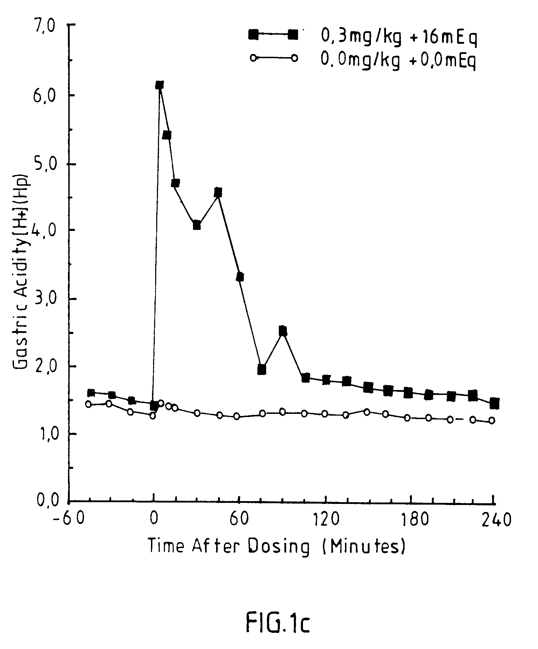

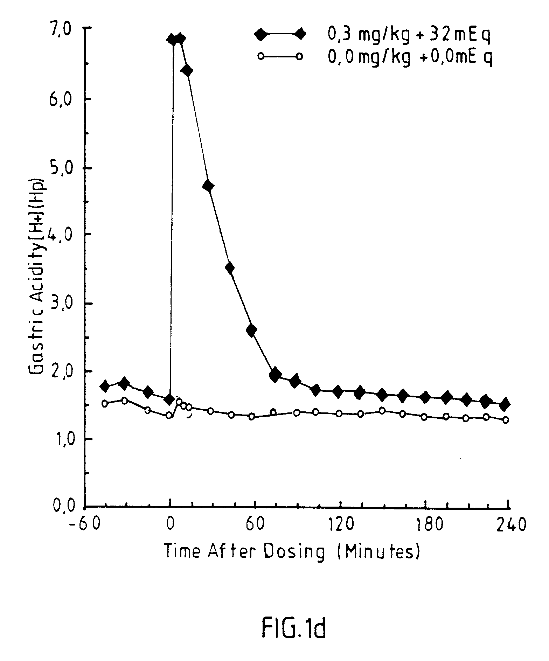

The Figures 1a-1d show the influence of an effervescent delivery system plus 0.3 mg/kg ranitidine upon gastric pH of dimiprit treated dogs with gastric fistulas versus time in minutes after dosing. Graphs compare data on mean pH of the gastric content in dogs pretreated with dimiprit and receiving water (open circles); receiving 0.3 mg/kg ranitidine alone (shaded circles, A); and receiving 0.3 mg/kg ranitidine in combination with effervescent components having different acid neutralizing capacities (8 mEq solid triangles, 16 mEq solid boxes, and 32 mEq solid diamonds, shown in Figures B, C and D, respectively).

Detailed Description of the Invention

It has now been found that the combination of an effervescent delivery system and a water soluble $H_2$ Blocker are synergistic such that the administration of the effervescent $H_2$ Blocker formulation provides continuity of antacid action for a reasonable period of time (1 to 3 hours). Instead of the immediate effect and short duration of a effervescent delivery system (citrate/bicarbonate solution) and the much delayed effect and long duration of the administration of $H_2$ Blocker alone; the solution of effervescent $H_2$ Blocker unexpectedly raises the pH of the gastric contents immediately and maintains the increased pH from about one to about three hours. In addition, it has been found that with an effervescent $H_2$ Blocker formulation, the dose of $H_2$ Blocker may be significantly reduced from the dosage commonly prescribed for twice daily treatment of duodenal ulcer.

The use of the effervescent delivery system permits the modification of the pharmacokinetics and pharmacodynamics of an $H_2$ Blocker resulting in a significant decrease in the time for onset of action of the antisecretory action of the $H_2$ Blocker while at the same time the effervescent delivery system also increases the potency and decreases the overall duration of action of the $H_2$ Blocker. The result is a formulation which requires less than about twenty-five percent of the $H_2$ Blocker dosage currently prescribed twice daily for the treatment of active duodenal ulcer. Such a formulation is particularly suitable for over-the-counter use for treatment of upset stomach associated with heartburn, sour stomach and acid indigestion by self-medicating consumers.

These findings are unexpected and significant for a number of reasons. $H_2$ Blockers inhibit gastric acid secretion and are prescribed for medical conditions such as active duodenal ulcer which require prolonged cessation of such secretion. The inhibition of gastric acid secretion by $H_2$ Blockers would theoretically provide relief of upset stomach associated with heartburn, sour stomach or acid indigestion. However, since the onset of action of $H_2$ Blockers is generally delayed one to two hours (or longer) after ingestion, such medication would not relieve the symptoms of upset stomach within a reasonable timeframe. In addition, the duration of action of $H_2$ Blockers at the currently recommended therapeutic dose is far longer than necessary to relieve the short term, self-limiting conditions of upset stomach resulting from heartburn, sour stomach or acid indigestion.

Accordingly, there would have been no basis on which to expect that $H_2$ Blockers would be particularly advantageous as over-the-counter (OTC) products for treatment of upset stomach associated with heartburn, sour stomach or acid indigestion, especially at the prescription dose levels currently used for treatment of duodenal ulcer and other pathological hypersecretory conditions. It is now possible to provide the delivery of a low dose of $H_2$ Blocker with a faster onset and shorter, but continuous, duration of action, suitable for the treatment of upset stomach associated with heartburn, sour stomach or acid indigestion. The present invention provides a means for delivering a dosage which will relieve such symptoms of upset stomach quickly and for the necessary duration for self-medication by consumers.

Any water soluble or water solubilizable compound which acts to inhibit gastric acid secretion by blocking the histamine $H_2$ receptor can be used. Suitable $H_2$ Blockers include, without limitation, the commercially available compounds, cimetidine (TAGAMET®, sold by SmithKline Beecham), famotidine (PEPCID®, sold by Merck, Sharp & Dohme), ranitidine (ZANTAC®, sold by Glaxo), nizatidine (AXID®, sold by Eli Lilly) as well as others reported in the literature such as etintidine, lupitidine, mifentidine, niperotidine, roxatidine, sufotidine, tuvatidine and zaltidine.

The phrase "an effervescent delivery system" refers to two component effervescent systems which are well known in the art, such as an alkali metal bicarbonate and citric acid. The delivery system may be formulated to obtain the desired ANC with well known techniques. Useful effervescent delivery systems can have an acid neutralizing capacity (ANC) of from about 3 to about 40 mEq. A preferred ANC is from about 4 to about 32 mEq.

Other components may also be included in an effervescent $H_2$ Blocker formulation as required or desirable for tableting and taste as are well known in the art.

A composition containing an effervescent delivery system and less than about 25 percent of the prescribed dosage of $H_2$ Blocker for twice daily treatment of active duodenal ulcer is suitable for self-medication for relief of upset stomach associated with heartburn, sour stomach or acid indigestion. This significant reduction in dosage will result in a reduction in the incidence and/or intensity of side-effects of $H_2$ Blockers, such reduction making the composition suitable for self-medication without a physician's supervision over extended periods of time. The reduced dosage will also reduce the potential for adverse interactions of the $H_2$ Blocker with other drugs which an individual may be taking.

For illustration, the four $H_2$ Blockers marketed in the United States as prescription medications are shown below with the oral dosage level suitable for twice daily treatment of active duodenal ulcer.

| cimetidine | 400 mg |
|---|---|
| ranitidine | 150 mg |
| nizatidine | 150 mg |
| famotidine | 20 mg |

If administered in an effervescent delivery system, the dosage of the above indicated $H_2$ Blocker may be less than about

| (effervescent) cimetidine | 100 mg |
|---|---|
| (effervescent) ranitidine | 37.5 mg |
| (effervescent) nizatidine | 37.5 mg |
| (effervescent) famotidine | 5 mg |

Preferred dosages are from about 5 to 20% of the current prescription dosage for twice daily treatment of active duodenal ulcer; more preferably 10 to 20%.

Other $H_2$ Blockers may be used in about the same proportional dosage, once a recommended therapeutic dosage for treatment of duodenal ulcer on a twice daily basis is established.

The formulation may be provided in the form of a sachet (a loose, granulated powder) or a tablet. Two tablets are commonly thought of as a single dose of an effervescent formulation and therefore the low dose of $H_2$ Blocker could be apportioned between two tablets.

The above indicated amounts of $H_2$ Blocker in an effervescent dosage form essentially represent an optimal range for duration of action, onset and potency of these compounds when administered for the treatment of the symptoms of upset stomach associated with heartburn, sour stomach or acid indigestion. Such an effervescent formulation would be self-medicated by the consumer for the immediate reduction of such symptoms and extended alleviation of those symptoms for approximately one to three hours.

By modifying the acid neutralizing capacity (ANC) of the effervescent delivery system (the effervescent couple) within an optimal range of approximately 4 to 32 mEq (ANC of 4 to 32) and by modifying the dosage of $H_2$ Blocker between about 25% and 5%, formulations of various degrees of antacid potency and duration of action can be provided while maintaining the immediate onset of action, continuous antacid action and significantly reduced dosage.

Preclinical studies in dogs with gastric fistulas (disclosed in the Example) clearly document that the effervescent delivery system provides an efficient vehicle for administration of $H_2$ Blockers which over-comes the problems of delayed onset of action, high dosage level and long duration of action that are common to $H_2$ Blockers. Instead of a short-lived immediate increase in pH associated with the antacid effect of the effervescent delivery system and a much delayed increase in pH from the $H_2$ Blocker (as would have been expected if the two were administered separately), an effervescent $H_2$ Blocker formulation provides continuity of acid neutralizing capacity. With the effervescent formulation, the short term (30 minutes) effect of the effervescent base and the longer onset time (2 hours) of the $H_2$ Blocker are essentially combined into a single effect in which the gap between antacid activity of the effervescent delivery system and onset of action of $H_2$ Blocker activity are unexpectedly combined to provide continuous action. The antacid continuity of such a composition can maintain the stomach pH above 3.5 as required in the OTC monograph for an antacid. The composition provides almost immediate onset of action and a duration of action generally not greater than about three hours with a significantly reduced dosage of $H_2$ Blocker than currently prescribed for medical conditions such as active duodenal ulcer. The delivery system is sufficient to provide immediate and continuous relief for ordinary occurrences of upset stomach associated with heartburn, sour stomach or acid indigestion without unnecessary overmedication.

The above indicates that an effervescent delivery system when coupled with an $H_2$ Blocker produces a product profile with clear advantages for treatment of upset stomach associated with heartburn, sour stomach or acid indigestion.

The following example shows the effect of an effervescent formulation of ranitidine on the gastric pH of a fistulated dog. Similar preliminary studies have been run with famotidine and cimetidine with similar findings. The example and the Figure disclose one embodiment of the invention, but do not limit the applicability of the invention which is solely defined by the claims.

Example

Studies were run with between 11 and 16 dogs as subjects. The data was collected on dogs with gastric fistulas which were treated with dimiprit to decrease gastric pH and simulate the acidity of a human stomach during an attack of "upset stomach" versus time in minutes. An amount of dimiprit, which is sufficient to maintain gastric acidity at pH of approximately 1.5, when no antacid medication is administered, was given to all the dogs by intravenous infusion throughout the studies. This treatment and pH should be assumed in all experiments presented in the Figure. Compositions of interest were tested for effect on gastric pH against this background pH.

The hypothesis that an effervescent delivery form combined with the $H_2$ Blocker would produce the desired antacid effect at a much lower dose of $H_2$ Blocker than when an $H_2$ Blocker is administered alone was tested. The data obtained on the model system using dogs with gastric fistulas by administering 0.3 mg/kg ranitidine without an effervescent delivery system is graphed in the Figure at A. It is important to note that the dose of 0.3 mg/kg is comparable to approximately twenty percent of the normal prescription dose of ranitidine used for treatment of humans with active duodenal ulcer. Figure A shows this low a dose of ranitidine, administered without the effervescent delivery system, does not affect the gastric pH. However, when this "ineffective" dose of ranitidine is administered in combination with the effervescent delivery system, the kinetics of ranitidine are modified significantly resulting in rapid onset of and increased potency of the antisecretory effect of ranitidine so that the effervescent formulation of $H_2$ Blocker produces immediate and continued antacid action (Figures B, C and D). The effect is both greater potency, and a longer and continuous duration of action than observed either with the administration of the effervescent base alone or with the administration of a 0.3 mg/kg dosage of ranitidine without the effervescent base. Data was collected with combinations of effervescent couples of different ANC (8 mEq. shaded triangles, 16 mEq. shaded boxes and 32 mEq. shaded diamonds) and is shown in the Figure at B, C and D respectively. This data indicates that the duration and potency of the combination may be affected by varying the ANC of the formulation.

The administration of dimiprit (a histamine agonist) given to dogs with gastric fistula, stimulates gastric acid secretion far in excess of normal human gastric acid secretion during an episode of upset stomach. It is recognized that the duration of action would be much longer (1 to 3 hours) in a human episode of upset stomach than that seen in the dog model because humans would not produce the maximal level of acidity seen in dogs treated with dimiprit.

**Claims**

1. A composition for treating an upset stomach associated with heartburn, sour stomach or acid indigestion, comprising:
   a. a water soluble $H_2$ Blocker, and
   b. an effervescent delivery system,
   the amount of $H_2$ Blocker being less than about twenty-five percent of the dosage prescribed for twice daily treatment of active duodenal ulcer; the composition providing immediate and continuous relief for from about one to about three hours when dissolved in water and administered orally.

2. The composition of Claim 1, the amount of $H_2$ Blocker being between about five and about twenty percent of the dosage prescribed for twice daily treatment of active duodenal ulcer.

3. The composition of claim 1 wherein the $H_2$ Blocker is chosen from the group consisting of ranitidine, famotidine, cimetidine or nizatidine.

4. The composition of claim 1 wherein the acid neutralizing capacity of the effervescent delivery system is between about 3 and about 40 milliequivalents.

5. The composition of claim 1 in tablet form.

FIG.1a

FIG.1b

FIG.1c

FIG.1d

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | FR-A-2 648 710 (LABORATORIES GLAXO S.p.A.)<br>* The whole document *<br>--- | 1-5 | A 61 K 31/34<br>A 61 K 9/00 |
| D,X | FR-A-2 593 065 (LABORATOIRES SMITH, KLINE & FRENCH)<br>* The whole document *<br>--- | 1-5 | |
| D,X | GB-A-2 219 940 (LABORATORIES GLAXO S.A.)<br>* The whole document *<br>----- | 1-5 | |

TECHNICAL FIELDS
SEARCHED (Int. Cl.5)

A 61 K

The present search report has been drawn up for all claims

| Place of search | Date of completion of the search | Examiner |
|---|---|---|
| THE HAGUE | 06-02-1992 | VENTURA AMAT A. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P0401)